# EUROPEAN PATENT SPECIFICATION

(11) **EP 3 795 503 B1**
(45) Date of publication and mention of the grant of the patent: **04.05.2022**
(21) Application number: 20195038.3
(22) Date of filing: 08.09.2020
(51) Int. Cl.: B65G 37/02, A61F 13/15, B65B 65/00, B65G 54/02, G05B 19/418

(54) **A MOVABLE DEVICE AND PACKAGING PLANT**
EINE BEWEGLICHE VORRICHTUNG UND VERPACKUNGSANLAGE
UN DISPOSITIF MOBILE ET UNE INSTALLATION D'EMBALLAGE

(30) Priority: 17.09.2019 IT 201900016427
(43) Date of publication of application: 24.03.2021
(73) Proprietor: Fameccanica.Data S.p.A., 66020 San Giovanni Teatino (CH) (IT)
(72) Inventor: SABLONE, Gabriele, I-65125 Pescara (IT)
(74) Representative: Marchitelli, Mauro

(56) References cited:
- EP-A1- 3 255 764
- KR-A- 20090 020 783
- US-A1- 2004 235 397
- US-A1- 2014 230 660
- US-A1- 2018 074 478

## Description

### APPLICATION FIELD

Embodiments of the present invention relate to a movable device for transporting groups of articles in a packaging plant wherein said articles are made and packaged.

Embodiments of the present invention also relate to a packaging plant comprising a plurality of movable devices.

Here and in the following description, for the sole purpose of illustration and non-limitative to this, the invention will be described with reference to absorbent sanitary articles, such as, for example, diapers, diaper-pants, incontinence pads, sanitary towels, or other articles intended to absorb body fluids. Although the description refers to absorbent sanitary articles, it is understood that the present invention can also be implemented to transport other types of articles, such as, for example, containers, bottles, books, magazines, objects, other articles and their combinations.

### STATE OF THE ART

There are transport systems configured to transport a plurality of articles from a production machine to a packaging machine.

Some known transport systems consist of fixed elements functionally connected to a production machine and to a packaging machine in a receiving area and in a delivery area, respectively.

The articles made by the production machine are supplied to the transport system in the receiving area so that these articles are transferred to the packaging machine along a fixed and preset path defined by the fixed elements.

These fixed elements may also be very bulky and can also hinder operators during the production and packaging of the articles.

If it is necessary to reposition the production machine and/or the packaging machine in the packaging plant, these transport systems need to be disassembled and reassembled in the new position.

This entails considerable losses from an economic point of view and also involves long interruptions in production.

It is known that the articles are transferred sequentially along a predefined path until they are delivered to the packaging machine while maintaining the same initial delivery order.

To change the delivery order of the articles, it is known to use multiple additional components that are subject to frequent hitches and require a significant slowdown in the operating cycle of the packaging plant.

Some of the known solutions allow removing some articles deemed to be defective without being able to intervene on the delivery order, let alone inserting other articles between one article and another.

It is known that known transfer systems allow only one type of article to be processed and typically do not allow the number of articles to be packaged to be modified without appropriate modifications being made while production is stopped.

In the large distribution sector and, in particular, in that of absorbent sanitary articles, there is a need to create packages with a desired number and/or type of groups of absorbent sanitary articles.

By virtue of the market needs and the variety of articles available, these groups may comprise an equal or different number of articles and, moreover, they may comprise identical or different articles.

For example, a first group of articles may include two incontinence pads, a bottled detergent, children's toys; and a second group of articles may include twenty diapers and ten toothbrushes.

Those skilled in the sector are unable to meet the needs of consumers on a large scale, as efficient methods and apparatuses are not known for producing customized packages comprising groups of articles that differ every time according to the demands of the various consumers.

The solutions known in the field have not yet made it possible to process and package a plurality of combinations of groups of articles quickly.

Some known solutions envisage the packaging of a fixed number of groups of articles of the same type.

This makes it possible to only partially satisfy the demands of consumers who do not find customized packages with various groups of articles on the market.

One of the main problems encountered in known plants is that they comprise different production machines which may have very different production rates.

This means an efficient solution has not been provided that is able to coordinate the production of articles at different packaging rates.

If it is necessary to package groups of articles with different numbers and/or different types of articles supplied by different production machines, it is known that these activities are carried out offline, i.e. in a grouping station not connected to the production machines or to the packaging machines, by operators who manually group the articles in relation to the specific customized package to be created.

It is known that these solutions that involve the use of operators for manual packaging are slow and, moreover, are subject to multiple non-monitorable errors for which it is not possible to implement actions to prevent them from happening again.

Other known solutions require frequent modifications to the plant in order to process and package different combinations of groups of articles. This significantly increases costs and production times.

Other solutions envisage producing a plurality of articles and storing them in such a way as to be able to package them manually at subsequent times in relation to the customized packages requested by consumers.

These known solutions present considerable problems both from a storage point of view and from the point of view of maintaining the performance of the articles, since it is known that these articles may also remain in storage for a long time.

This entails high costs and considerable losses as the articles, deteriorating over time, can no longer be put on the market.

Other solutions involve the use of self-driving vehicles on which the articles are deposited and brought close to the packaging machine and then delivered to the packaging machine manually, or by means of special picking-up robots.

However, these known solutions are slow and not very flexible in a context wherein the needs of consumers are very diversified in terms of quantity of articles, and in terms of types. See, for example, what is described in documents US-A-2004/235397 and EP-A-3255764

Document US 2004/235397 A1 discloses a movable device suitable for transporting groups of articles in a packaging plant comprising at least one production machine of said groups of articles and at least one packaging machine configured to form a desired combination of said groups of articles, wherein said movable device comprises a frame provided with movement means suitable to move said frame in said packaging plant at least from said production machine to said packaging machine, wherein said movable device comprises a guide circuit coupled to said frame, and at least one transport unit comprising a body and a containment member connected to said body, wherein said containment member is suitable to contain at least one of said groups of articles. Document US 2018/074478 A1 discloses a packaging plant according to the preamble of claim 6.

There is a need to improve and make an effective solution available that allows managing a plurality of articles, even different from each other, in a packaging plant, which overcomes at least one of the drawbacks of the prior art.

One object of the present invention is to provide a device for transporting articles in a packaging plant which allows various and different groups of articles to be processed quickly in relation to the specific batches to be packaged.

It is also an object of the present invention to provide a device for transporting articles in a packaging plant which does not obstruct and hinder the operators.

Another object of the present invention is to provide an automatic and flexible packaging plant that allows creating various and different customized packages, without the need to interrupt production, and without the use of operators for manual packaging.

The present Applicant has devised, tested and implemented this invention to overcome the drawbacks of the prior art and to obtain these and additional objects and advantages.

### SUMMARY OF THE INVENTION

The present invention is expressed and characterized in the independent claims, while the dependent claims set out other characteristics of the present invention or variants of the idea of the main solution.

The invention provides a movable device according to claim 1 for transporting groups of articles in a packaging plant, comprising at least one production machine of groups of articles and at least one packaging machine configured to form a desired combination of groups of articles.

The movable device comprises a frame provided with movement means configured to move the frame in the packaging plant at least from the production machine to the packaging machine.

The movable device comprises a guide circuit coupled to the frame, and at least one transport unit comprising a body and a containment member connected to the body and configured to contain at least one of the groups of articles.

The body is provided with a first magnetic element configured to magnetically couple to the guide circuit, so that, during use, the body is in the desired position along the guide circuit.

The transport unit is configured to position itself in the desired position along the guide circuit.

The present invention allows groups of articles to be received from one or more production machines and to deliver them in the desired order to the desired packaging machines.

The present invention allows transporting various and different groups of articles, also of different types, and delivering them to the desired packaging machines so as to produce customized packages in relation to the specific needs of consumers.

Thanks to the movable device, it is possible to simultaneously move multiple groups of articles in the packaging plant, avoiding having to provide fixed transport systems functionally connected to both the production machine and the packaging machines provided in the packaging plant.

This allows obtaining a packaging plant wherein there are no bulky fixed transport systems, and wherein it is possible to reposition the production machine and the packaging machine.

In the latter case, the movable device is programmable so that it positions itself at the production machine and the packaging machine in relation to their current position in the packaging plant.

This is advantageous compared to known solutions that envisage fixed transport systems for which it is necessary to modify the entire set-up of the transport system if it is intended to change the position and/or type of production machine and packaging machine.

The transport units can be positioned along their respective guide circuits so that when the movable device is positioned near the production machine or the packaging machine, it is possible to receive and deliver the groups of articles in the correct transfer position.

The movable device can be substantially configured as an automated-guided vehicle, called AGV, capable of moving around the packaging plant.

The movable device is provided with one or more guide circuits on which a plurality of transport units can move and position themselves.

The transport unit is provided with a second magnetic element, opposite to the first magnetic element, configured to magnetically couple to another distinct guide circuit separate from the guide circuit and present in the packaging plant, or present on another movable device.

This allows the transport unit to pass from one movable device to another, or to pass to a guide circuit present in the packaging plant.

This allows transferring the desired transport units and consequently the desired groups of articles from one movable device to another, or to a guide circuit present in the packaging plant.

This makes the packaging plant extremely flexible, as it is possible to transport, transfer, group and deliver the desired groups of articles in the desired order and with coordinated and optimized timing so that the articles are packaged according to the desired needs of consumers, and quickly.

The first magnetic element and the second magnetic element are selectively activated so that the transport unit is coupled to the guide circuit, or to the other guide circuit at a coupling tract wherein, during use, the guide circuits are facing each other.

This allows the transport unit to be coupled to the desired guide circuit in the coupling tract, that is, during use, when two guide circuits are facing each other.

In this way, the transport unit can easily and quickly perform the exchange, or rather, it can pass from a guide circuit to another guide circuit coupled to it.

The coupling tract can also be defined temporarily if the movable device is positioned so that one of the guide circuits present on the device faces the other guide circuit present in the packaging plant, or present in another movable device.

According to possible embodiments, the movable device comprises at least two distinct guide circuits, separated and facing each other at respective coupling tracts.

In accordance with possible embodiments, the movable device may comprise a lifting member configured to bring the guide circuit from a first height to a second height, different from the first height.

This allows positioning the transport units at the desired height in order to perform the transfer of the groups of articles at the height of the transfer of the articles of the production machine and of the packaging machine.

According to possible embodiments, the movable device comprises a communication unit configured for receiving and transmitting a signal with a control and command unit configured to control the movable device and/or other movable devices present in the packaging plant.

The signal can include the command instruction of the movable device to be controlled.

The control and command unit may advantageously remotely control one or more movable devices so as to transport a plurality of articles in the desired order from the production machines to the packaging machines or to other guide circuits present in the packaging plant along a desired path.

In accordance with possible embodiments, the movable device may comprise a rechargeable electric power supply unit configured to power the movable device.

This allows movable devices to be arranged that are not connected to a fixed electric power supply, except to recharge the rechargeable electric power supply unit.

The present invention also relates to a packaging plant of groups of articles according to claim 6, comprising at least one production machine of groups of articles and at least one packaging machine configured to form a desired combination of groups of articles.

The packaging plant comprises a plurality of movable devices as in any of the possible embodiments.

This packaging plant is highly flexible and automated, since it is possible to create multiple combinations of articles by controlling the plurality of movable devices in a coordinated manner.

In accordance with possible embodiments, the packaging plant comprises a guide circuit functionally coupled to at least two packaging machines, so that, during use, the transport units can be positioned at any of the at least two packaging machines.

This allows one or more movable devices to position themselves simultaneously so that the respective guide circuits face the guide circuit connected to the two packaging machines, and that the transport units pass to this latter guide circuit in order to move to the desired packaging machine to deliver the desired articles.

According to possible embodiments, the packaging plant may comprise a guide circuit functionally coupled to at least one production machine, so that, during use, the transport units can be positioned at the production machine.

This makes it possible to maintain production continuity as the production machine can supply the articles continuously to the transport units present in the guide circuit functionally coupled thereto, without the need for a ready movable device, with the transport unit transported thereby, placed at the receiving area to transfer the articles from the production machine to the transport unit.

This allows the movable devices to be positioned so that the respective guide circuits face the guide circuit connected to the production machine simultaneously or in sequence, and subsequently move towards the desired packaging machine to deliver the desired articles, without there being the need to interrupt the production of the production machine in the time interval between the positioning of one movable device and the next.

According to possible embodiments, the packaging plant comprises a control unit configured to control the plurality of movable devices in a coordinated manner so as to form the desired combination of groups of articles in the packaging machine.

This allows setting the movement of the movable devices each time so that they transport the articles in the desired order to the packaging machines in a coordinated way, or rather, so that the movable devices do not hinder each other.

In accordance with possible embodiments, the packaging plant 100 comprises a charging station wherein there is at least one charging device configured to recharge one or more rechargeable electric power supply units of the movable devices.

This makes it possible to optimize the arrangement of the components of the packaging plant so that when one or more movable devices are about to run out of charge in their respective rechargeable electric power supply units, they can be directed towards the charging station.

The charging station may be advantageously placed outside the maneuvering space of the packaging plant between the production machines and the packaging machines so that the movable devices are not in the way while they are in the charging station.

### ILLUSTRATION OF THE DRAWINGS

These and other characteristics of the present invention will become clear from the following description of embodiments, given as a non-limiting example, with reference to the attached drawings wherein:
- Figure 1 schematically illustrates a packaging plant according to a possible embodiment of the present invention;
- Figure 2 schematically illustrates a detail of a transport apparatus according to a possible embodiment of the present invention;
- Figure 3 schematically illustrates a transport unit according to a possible embodiment of the present invention;
- Figures 4 and 5 schematically illustrate two details of two possible embodiments of a guide circuit according to the present invention;
- Figure 6 schematically illustrates a packaging plant according to a possible embodiment of the present invention;
- Figures 7-12 schematically illustrate possible embodiments of a transport apparatus according to the present invention;
- Figure 13 schematically illustrates a detail of a guide circuit according to a possible embodiment of the present invention;
- Figure 14 schematically illustrates a packaging plant according to a possible embodiment of the present invention;
- Figures 15 and 16 schematically illustrate two possible embodiments of a movable device according to the present invention.

To facilitate understanding, identical reference numbers have been used, where possible, to identify identical common elements in the Figures.

### DESCRIPTION OF THE EMBODIMENTS

The invention is defined by the independent claims. Embodiments of the invention become apparent from the dependent claims.

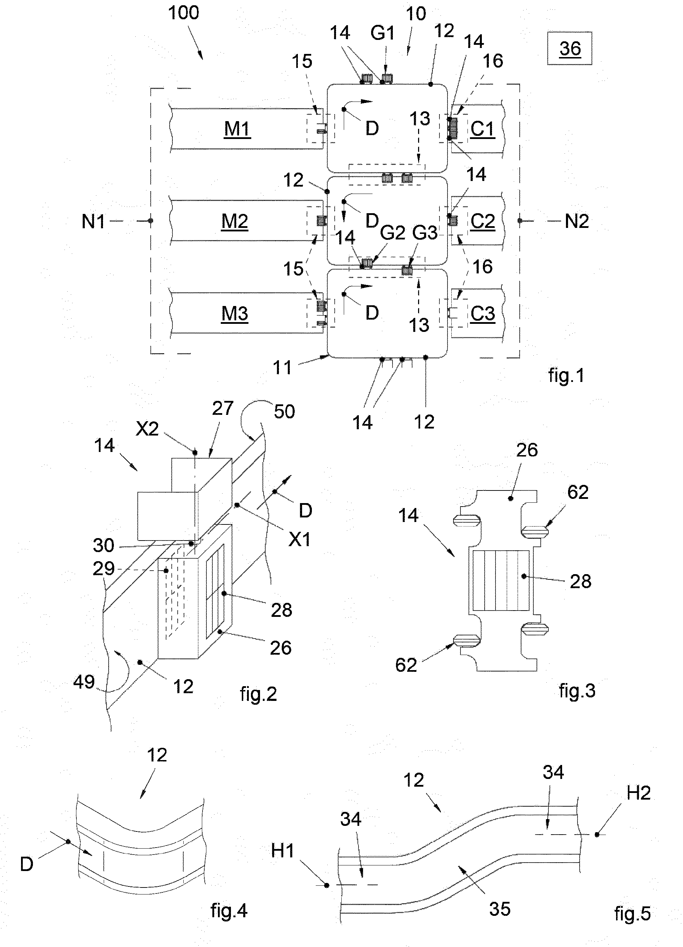

Embodiments described here, with reference to Figures 1-6 and others, refer to a packaging plant 100 of groups G1, G2, G3 of absorbent sanitary articles including some details and to a method for managing a packaging plant 100.

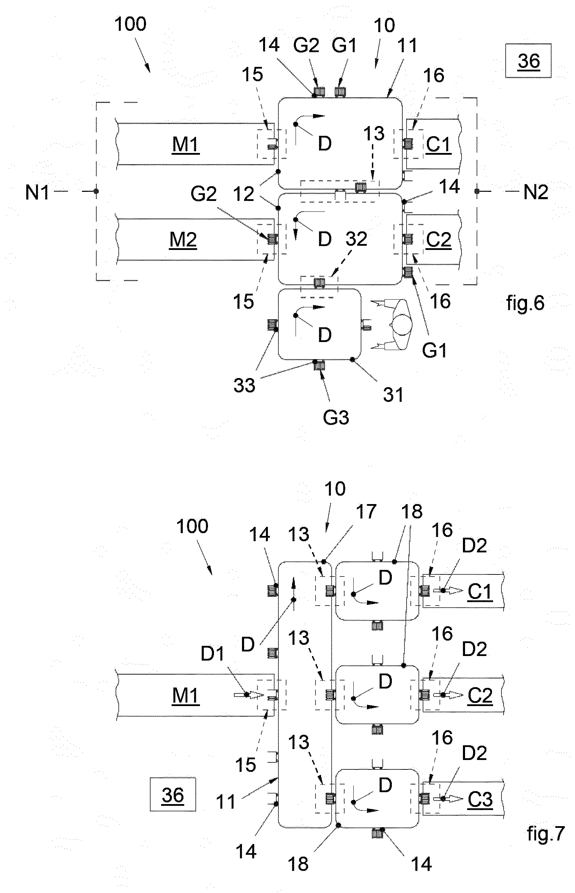

Additional embodiments described here, with reference to Figures 7-9 and others, refer to a transport apparatus 10 for absorbent sanitary articles and to a method for transporting relative absorbent sanitary articles.

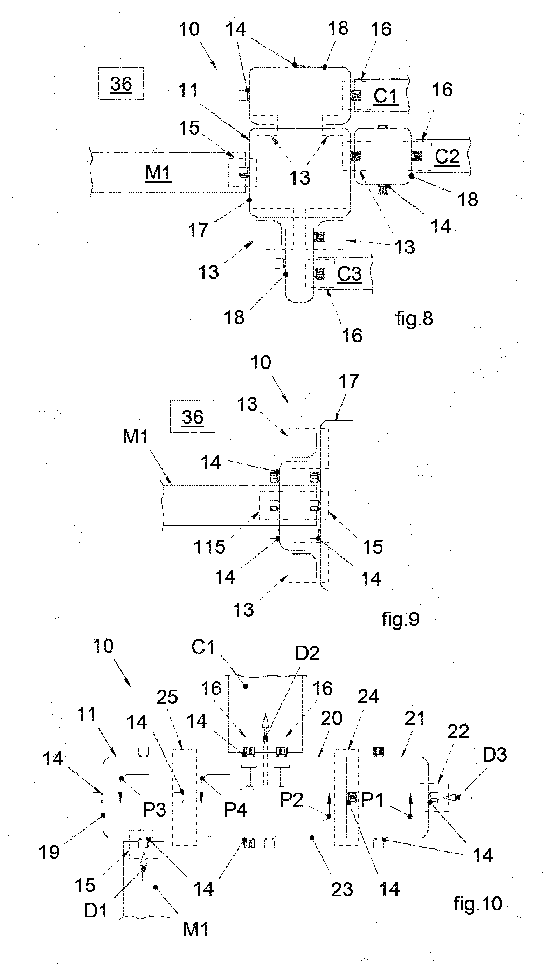

Other embodiments described here, with reference to Figures 10-12 and others, refer to an apparatus 10 for transporting absorbent sanitary articles suitably designed to manage possible insertions of absorbent sanitary articles coming from non-compliant packages, or of particular absorbent sanitary articles, or other articles to be combined with already formed groups of absorbent sanitary articles.

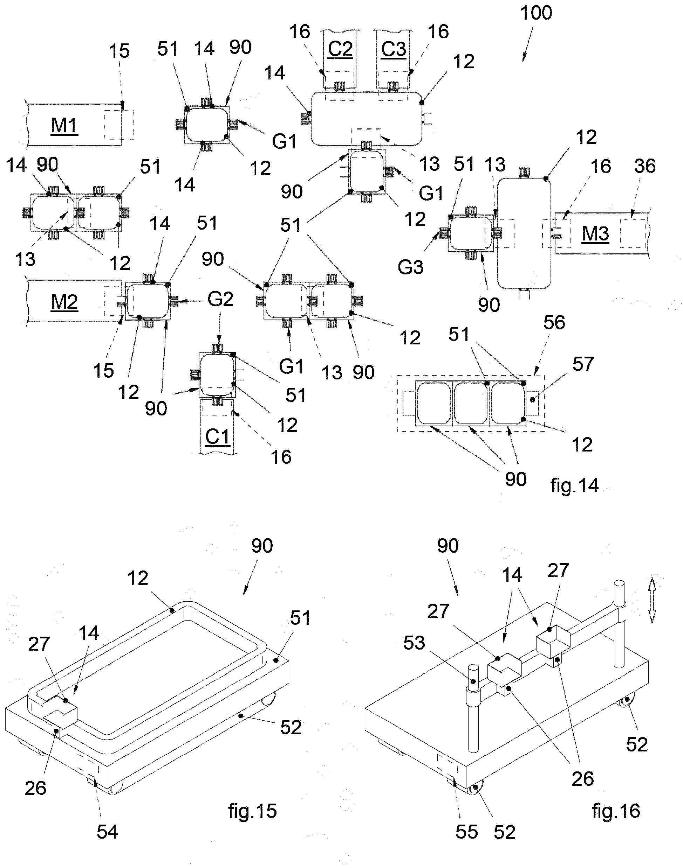

Embodiments described here, with reference to Figures 14-16 and others, refer to a movable device 90 for transporting groups G1, G2, G3 of articles in a packaging plant 100, and to a packaging plant 100 comprising a plurality of these movable devices 90.

The packaging plant 100 of groups G1, G2, G3 of absorbent sanitary articles comprises a first number N1 of production machines M1, M2, M3 of groups G1, G2, G3 of absorbent sanitary articles.

In accordance with possible embodiments, the production machines M1, M2, M3 may be configured to produce the same or different types of absorbent sanitary articles.

According to possible embodiments, one or more production machines M1, M2, M3 can be configured to produce absorbent sanitary articles with different or identical properties and/or components.

In accordance with possible embodiments, at least one of the production machines M1, M2, M3 may be configured to produce groups G1, G2, G3 of absorbent sanitary articles of one type and at least one other of the production machines M1, M2, M3 may be configured to produce groups G1, G2, G3 of absorbent sanitary articles of another type.

For example, the two groups of absorbent sanitary articles of different types may have two different sizes.

According to possible embodiments, at least two production machines M1, M2, M3 may be arranged in the packaging plant 100 so that the release of the absorbent sanitary articles is oriented towards a common release direction.

In accordance with possible embodiments, at least two production machines M1, M2, M3 may be arranged in the packaging plant 100 so that the release of the absorbent sanitary articles is oriented in the opposite direction towards a common area of the packaging plant 100.

In accordance with possible embodiments, at least two production machines M1, M2, M3 may be arranged in the packaging plant 100 so that the respective release directions of the absorbent sanitary articles are perpendicular to each other towards a common area of the packaging plant 100.

In accordance with possible embodiments, at least two production machines M1, M2, M3 may be configured to supply the absorbent sanitary articles at different heights, wherein each height is defined with respect to a common horizontal reference.

For example, if two production machines M1, M2, M3 are arranged at different areas of the packaging plant 100, wherein the respective support surfaces have a difference in height, the height of each production machine M1, M2, M3 at which absorbent sanitary articles are supplied is defined, for example, in relation to the support surface of one of the two production machines M1, M2, M3.

In accordance with possible embodiments, at least two production machines M1, M2, M3 may be configured to supply the absorbent sanitary articles at the same height, wherein each height is defined with respect to a common horizontal reference.

According to possible embodiments, the first number N1 is equal to or greater than one.

The packaging plant 100 comprises a second number N2 of packaging machines C1, C2, C3 of groups G1, G2, G3 of absorbent sanitary articles.

The packaging machines C1, C2, C3 are configured to form a desired combination of groups G1, G2, G3 of absorbent sanitary articles.

The combination may include groups G1, G2, G3 of identical or different absorbent sanitary articles, both from the point of view of the number of absorbent sanitary articles present in the group G1, G2, G3, and from the point of view of the type of absorbent sanitary articles in the same group G1, G2, G3, or between distinct groups G1, G2, G3, as well as from the combined point of view of number and type.

According to possible embodiments, the packaging machine C1, C2, C3 may be configured to produce a customized package containing the desired combination.

In accordance with possible embodiments, the customized package may comprise a flexible packaging, a rigid cardboard, or other type of flexible or rigid packaging, in relation to the packaging and transport requirements.

According to possible embodiments, at least two packaging machines C1, C2, C3 may be arranged in the packaging plant 100 so that the receiving of groups G1, G2, G3 of absorbent sanitary articles is oriented towards a common receiving direction.

According to possible embodiments, at least two packaging machines C1, C2, C3 may be arranged in the packaging plant 100 so that the receiving of groups G1, G2, G3 of absorbent sanitary articles is oriented in the opposite direction towards a common area of the packaging plant 100.

According to possible embodiments, at least two packaging machines C1, C2, C3 may be arranged in the packaging plant 100 so that the respective directions of receiving of the groups G1, G2, G3 of absorbent sanitary articles are perpendicular between each other towards a common area of the packaging plant 100.

In accordance with possible embodiments, at least two packaging machines C1, C2, C3 can be configured to receive the groups G1, G2, G3 of absorbent sanitary articles at different heights, wherein each height is defined with respect to a common horizontal reference.

For example, if two packaging machines C1, C2, C3 are arranged at different areas of the packaging plant 100 wherein the respective support surfaces have a difference in height, the height of each packaging machine C1, C2, C3 at which the groups G1, G2, G3 of absorbent sanitary articles are received is defined, for example, in relation to the support surface of one of the two packaging machines M1, M2, M3.

In accordance with possible embodiments, at least two packaging machines C1, C2, C3 may be configured to receive the groups G1, G2, G3 of absorbent sanitary articles at a common height, wherein each common height is defined with respect to a common horizontal reference.

In accordance with possible embodiments, at least one production machine M1, M2, M3 and one packaging machine C1, C2, C3 may be configured, respectively, to supply and to receive the groups G1, G2, G3 of absorbent sanitary articles at different heights, wherein each height is defined with respect to a common horizontal reference.

In accordance with possible embodiments, at least one production machine M1, M2, M3 and one packaging machine C1, C2, C3 may be configured, respectively, to supply and to receive the groups G1, G2, G3 of absorbent sanitary articles at a common height, wherein each common height is defined with respect to a common horizontal reference.

According to possible embodiments, the second number N2 is equal to or greater than two.

The packaging plant 100 comprises a transport apparatus 10 functionally connected to the first number N1 of production machines M1, M2, M3 and to the second number N2 of packaging machines C1, C2, C3.

Here and below, "functional connection" means a connection between two components of the packaging plant 100 configured to create a continuous process of production, transport and packaging of groups G1, G2, G3 of absorbent sanitary articles.

The functional connection can be produced by suitable transfer means configured to transfer and to form groups G1, G2, G3 of absorbent sanitary articles. For example, the transfer means may comprise pushers, manipulators, robots, conveyor belts, or the like.

In accordance with possible embodiments, each of the one or more production machines M1, M2, M3 is functionally connected to the transport apparatus 10 at respective receiving areas 15, wherein the absorbent sanitary articles produced by the respective production machine M1, M2, M3 are supplied.

According to possible embodiments, each of the packaging machines C1, C2, C3 is functionally connected to the transport apparatus 10 at respective delivery areas 16, wherein the absorbent sanitary articles are delivered to the respective packaging machine C1, C2, C3.

In accordance with possible embodiments, the transport apparatus 10 is configured to transport the groups G1, G2, G3 of absorbent sanitary articles from the production machine(s) M1, M2, M3 to the packaging machines C1, C2, C3 in the desired order.

The transport apparatus 10 comprises a guide assembly 11 and a plurality of transport units 14.

The guide assembly 11 is provided with at least two distinct guide circuits 12, separated and facing each other at least in one coupling tract 13.

According to possible embodiments, each of the guide circuits 12 can be shaped so as to each define its own direction of movement D.

In accordance with possible embodiments, in the coupling portion 13 the guide circuits 12 facing each other have their respective directions of movement D substantially parallel to each other.

According to possible embodiments, at least one of the guide circuits 12 is functionally connected to at least one production machine M1, M2, M3 by means of at least one receiving area 15.

According to possible embodiments, at least one of the guide circuits 12 is functionally connected to at least one packaging machine C1, C2, C3 by means of at least one delivery area 16.

In accordance with possible embodiments, the guide circuit 12 may comprise a plurality of elements connected together to form the desired path.

In accordance with possible embodiments, at least one of the guide circuits 12 may define a closed path.

According to possible embodiments, at least one of the guide circuits 12 may define an open path.

According to possible embodiments, the coupling between at least two guide circuits 12 may be obtained by means of a distinct intermediate coupling element, separated and facing a guide circuit 12 in a first intermediate coupling tract and to another guide circuit 12 in a second intermediate coupling tract.

In accordance with possible embodiments, the coupling between at least two guide circuits 12 may be obtained by means of a plurality of intermediate coupling elements coupled together, wherein at least one intermediate coupling element is distinct, separated and facing a guide circuit 12 in an intermediate coupling tract, and at least one other intermediate coupling element is distinct, separated and facing another guide circuit 12 in another intermediate coupling tract.

According to possible embodiments, the elements present in the guide circuit 12 may have a solid shape having two surfaces 49 and 50 opposite each other and configured to make the coupling with the transport units 14.

In accordance with possible embodiments, the elements present in the guide circuit 12 may have a solid shape which extends along the direction of movement D.

According to possible embodiments, at least part of the elements present in the guide circuit 12 may have a solid shape defining a linear tract.

According to possible embodiments, at least part of the elements present in the guide circuit 12 may have a solid shape defining a curved tract.

In accordance with possible embodiments, at least one of the guide circuits 12 may comprise at least two guide portions 34, located at different heights H1 and H2 and connected to each other by means of a connecting portion 35.

In accordance with possible embodiments, the heights H1 and H2 relate to a common geometric plane of horizontal reference. For example, the geometric reference plane may coincide with the ground.

According to possible embodiments, the transport unit 14 may be configured to pass from a guide portion 34 to another guide portion 34 along the connecting portion 35.

In accordance with possible embodiments, the guide circuit 12 may comprise at least one spiral tract 48 wherein there is a first surface 49 and a second surface 50, opposite to the first surface 49, arranged along a spiral path, so that upon leaving the spiral path the first surface 49 and the second surface 50 are inverted with each other.

In accordance with possible embodiments, each of the transport units 14 can be configured to transport at least the groups G1, G2, G3 of absorbent sanitary articles along a desired path defined by the guide circuits 12.

For example, the desired path may comprise tracts of one or more guide circuits 12, even repeated so that a transport unit 14 travels the same tract of the guide circuit 12 several times.

According to possible embodiments, each transport unit 14 is configured to move along the desired path independently from the other transport units 14.

According to possible embodiments, each transport unit 14 is configured to stop at a guide circuit 12 to which it is coupled.

According to possible embodiments, the movement and/or stopping along the guide circuit 12 may be obtained by magnetic movement means, aerostatic movement means, combinations thereof, or other means. For example, the movement can be substantially obtained as in linear magnetic motors.

According to possible embodiments, two or more transport units 14 may be configured to jointly transport one or more groups G1, G2, G3 of absorbent sanitary articles.

According to possible embodiments, a first transport unit 14 may be configured to transport a group G1, G2, G3 of absorbent sanitary articles and a second transport unit 14 may be configured to proceed at least for a tract along the same path followed by the first transport unit 14.

In this exemplary and non-limiting case, the second transport unit 14 may comprise retaining means configured to hold the group G1, G2, G3 of absorbent sanitary articles in position on the first transport unit 14.

For example, the retaining means of the second transport unit 14 may comprise a plate, pincers, or other retaining elements which can be positioned at the first transport unit 14 so as to prevent the groups G1, G2, G3 from falling from the first transport unit 14.

According to possible embodiments, two or more transport units 14 may be configured to proceed in a coordinated manner at least for a tract along the same path of the guide assembly 11.

According to possible embodiments, each of the transport units 14 is configured to pass from one of the guide circuits 12 to another guide circuit 12, and vice versa, at the coupling tract 13.

According to possible embodiments, each of the transport units 14 may comprise a body 26 and a containment member 27 connected to the body 26.

The containment member 27 is configured to contain at least one group G1, G2, G3 of absorbent sanitary articles.

The containment member 27 defines a containment compartment capable of containing a desired number of absorbent sanitary articles to form a group G1, G2, G3 of said articles.

In accordance with possible embodiments, the containment member 27 may be configured to define the desired containment compartment each time.

According to possible embodiments, the containment member 27 may comprise containment walls defining the containment compartment.

In accordance with possible embodiments, the containment walls may be positioned in the desired position so as to define, each time, the size of the containment compartment and, therefore, the number of absorbent sanitary articles of the group G1, G2, G3 to be transported.

In accordance with possible embodiments, the containment member 27 may be provided with retaining means designed to hold the group G1, G2, G3 of absorbent sanitary articles in position.

According to possible embodiments, the containment member 27 may be provided with one or more sensors designed to detect one or more pieces of information relating to the group G1, G2, G3 of absorbent sanitary articles that it contains. For example, the sensors may detect the type, position, weight, number or other parameters relating to the individual absorbent sanitary articles and/or to the group G1, G2, G3.

According to possible embodiments, the containment member 27 may be connected to the body 26 by means of a connecting member 30 configured to rotate around a rotation axis X1 parallel to the direction of movement D defined by the guide circuit 12.

This allows rotation of the containment organ 27 around the rotation axis X1 and then to position it in the desired way with respect to the rotation axis X1.

According to possible embodiments, the transport unit 14 may comprise positioning means configured to position the containment member 27 with respect to the body 26.

For example, the positioning means may position the containment member 27 at a desired height.

According to possible embodiments, the connecting member 30 can be configured to rotate around a second rotation axis X2 perpendicular to the direction of movement D.

This allows orienting the containment organ towards the desired direction perpendicular to the second rotation axis X2.

The body 26 is provided with a first magnetic element 28 configured to magnetically couple to one of the guide circuits 12 and with a second magnetic element 29, opposite to the first magnetic element 28, configured to magnetically couple to another guide circuit 12.

The first magnetic element 28 and the second magnetic element 29 can be selectively activated so that the transport unit 14 is coupled to one of the guide circuits 12 in the coupling tract 13.

In accordance with possible embodiments, the first magnetic element 28 and/or the second magnetic element 29 may comprise one or more magnets.

According to possible embodiments, the magnets may be operated by means of a source of electrical energy.

In accordance with possible embodiments, each of the transport units 14 may be provided with movement means configured to move the transport unit 14 along the guide circuit 12 to which it is coupled.

For example, the magnetic elements 28 and 29 may also be configured to move the transport unit 14 along the guide circuit 12.

According to possible embodiments, the movement means are configured to be operated remotely, for example, from a terminal.

In accordance with possible embodiments, the movement means may be included in the guide circuit 12.

According to possible embodiments, the transport unit 14 is configured to stop at an area of the guide assembly 11.

In accordance with possible embodiments, the transport unit 14 may be provided with auxiliary movement means 62 joined to the guide circuit 12 to facilitate the guide and support of the transport unit 14 on the guide circuit 12.

For example, the auxiliary movement means 62 may comprise a pair of wheels spaced apart along the longitudinal development of the body 26 of the transport unit 14 which, while the transport unit 14 is coupled to a guide circuit 12, are configured to rotate along a guide path, not illustrated, defined by the coupling surface of the guide circuit 12.

In other words, once the pair of wheels 62 is coupled to the coupling surface, for example, by means of guide rails, or by means of suitable protrusions of the coupling surface, defining the guide path, the transport unit 14 is stably coupled to the guide circuit 12.

In accordance with possible embodiments, the guide assembly 11 may comprise at least one distinct auxiliary guide circuit 31, separated and facing one of the guide circuits 12 at least at a coupling tract 32, and at least one auxiliary transport unit 33.

According to possible embodiments, the auxiliary transport unit 33 can be configured substantially like the transport units 14.

In accordance with possible embodiments, the auxiliary transport units 33 may be configured to transport one of the groups G1, G2, G3 of absorbent sanitary articles and/or an additional article along a desired path defined by the guide circuits 12.

In accordance with possible embodiments, the auxiliary transport unit 33 is configured to pass from the auxiliary guide circuit 31 to one of the secondary guide circuits 12, and vice versa, at the auxiliary coupling tract 32.

In accordance with possible embodiments, the auxiliary transport unit 33 is configured to stop in the auxiliary guide circuit 31 and to proceed along the auxiliary guide circuit 31 at a speed lower than the speed of the auxiliary transport unit 33 in the guide circuits 12.

This allows the operator to act safely while interacting with the auxiliary transport unit 33, as the speed of the latter is limited or even zero in the auxiliary guide circuit 31.

According to possible embodiments, the transport unit 14 may be configured as the auxiliary transport unit 33 so as to be able to stop or proceed along the auxiliary guide circuit 31 at a lower speed than the transport unit 14 in the guide circuits 12.

The auxiliary guide circuit 31 is useful for inserting absorbent sanitary articles or other articles into the transport cycle to be joined to the groups G1, G2, G3 of absorbent sanitary articles in order to create further customized packages.

Thanks to the auxiliary guide circuit 31, it is possible to insert articles into the transport cycle without interrupting the production of the articles.

According to possible embodiments, the transport units 14 may be configured to pass from a guide circuit 12 to the auxiliary guide circuit 31 at the coupling tract 32.

In this way, the auxiliary guide circuit 31 may also be useful for carrying out maintenance operations on the transport units 14.

According to possible embodiments, the packaging plant 100 comprises a control and command unit configured to control the transport units 14 in a coordinated manner to bring the groups G1, G2, G3 of absorbent sanitary articles into the packaging machines C1, C2, C3 in the desired order in relation to the desired combination of the groups G1, G2, G3 of absorbent sanitary articles to be made in each of the packaging machines C1, C2, C3.

For example, the control and command unit 36 may be fixed to one of the production machines M1, M2, M3, to one of the packaging machines C1, C2, C3, to the guide assembly 11, or it can be a portable component. The control and command unit 36 may include any of the following components: a computer, circuitry, a tablet, a smartphone, a programmable controller, and their combinations.

According to possible embodiments, the control and command unit 36 is configured to receive a signal comprising information on the combinations of groups G1, G2, G3 to be created, to set the path to be covered, and the stop times for each transport unit 14, to bring the groups G1, G2, G3 into the desired order from the production machines M1, M2, M3 to the packaging machines C1, C2, C3.

In accordance with possible embodiments, the control and command unit 36 is configured to modify the paths and travel times in relation to an additional signal including information on a further combination of groups G1, G2, G3 that is to be created.

According to possible embodiments, the travel times and paths can be determined in relation to the specific production rates and the packaging times of the packaging machines C1, C2, C3.

In accordance with possible embodiments, the control and command unit 36 may be advantageously configured to receive signals from the individual transport units 14 and/or from sensors positioned in the packaging plant 100, so that it can control the transport units 14 in relation to the signals received without them hindering each other and in order to optimize the times and paths of each transport unit to bring the groups G1, G2, G3 of absorbent sanitary articles quickly to the packaging machines C1, C2, C3.

According to possible embodiments, the signals may comprise the position in the guide assembly 11 of the desired transport unit 14.

The following embodiments, which are not a part of the present invention, relate to a method for managing a packaging plant 100.

According to possible embodiments, the method comprises:
- a setting step of the supply order of the groups G1, G2, G3 of absorbent sanitary articles for each of said packaging machines C1, C2, C3;
- a supplying step of the groups G1, G2, G3 of absorbent sanitary articles from one or more production machines M1, M2, M3 to respective transport units 14;
- a transporting step of the groups G1, G2, G3 of absorbent sanitary articles by means of the transport units 14 towards the packaging machines C1, C2, C3;
- a delivery step of the groups G1, G2, G3 of absorbent sanitary articles to said packaging machines C1, C2, C3 according to the respective set supply order.

According to possible embodiments, the management method envisages that at least two of the production machines M1, M2, M3 supply groups G1, G2, G3 of absorbent sanitary articles of different numbers.

According to possible embodiments, the management method envisages that at least two of the production machines M1, M2, M3 supply groups G1, G2, G3 of absorbent sanitary articles of different types.

In accordance with possible embodiments, the apparatus 10 for transporting absorbent sanitary articles comprises a guide assembly 11 and a plurality of transport units 14.

According to possible embodiments, the guide assembly 11 may present at least one receiving area 15 functionally connected to a production machine M1 of absorbent sanitary articles, and a plurality of delivery areas 16, each functionally connected to a respective packaging machine C1, C2, C3.

According to possible embodiments, each transport unit 14 may be configured to receive absorbent sanitary articles from the production machine M1 in the receiving area 15 and to transport them to one of the delivery areas 16 along a desired path defined by the guide assembly 11.

In accordance with possible embodiments, the guide assembly 11 may comprise a primary guide circuit 17 wherein the receiving area 15 is present, and a plurality of secondary guide circuits 18, each provided with a respective delivery area 16 and each distinct, separated and facing the primary guide circuit 17 in a respective coupling tract 13.

In accordance with possible embodiments, the transport units may be configured to pass from the primary guide circuit 17 to one of the secondary guide circuit 18, and vice versa, at the coupling tract 13.

The transport apparatus 10 allows functionally connecting a production machine M1 of absorbent sanitary articles to multiple packaging machines C1, C2, C3 so as to be able to create even different combinations of absorbent sanitary articles, in relation to the needs of the consumers.

Thanks to the transport apparatus 10, it is possible to create customized packages with various combinations of absorbent sanitary articles in parallel and without interrupting the production flow of the production machine M1.

For example, it is possible to set one of the packaging machines C1, C2, C3 for producing a first package containing a first combination of absorbent sanitary articles and another of the packaging machines C1, C2, C3 for producing a second package containing a second combination of absorbent sanitary articles.

The transport apparatus 10 is considerably more advantageous than known solutions which envisage storing the absorbent sanitary articles to subsequently package them manually in relation to the orders received.

According to possible embodiments, the coupling between the primary guide circuit 17 and the secondary guide circuit 18 may be obtained by means of a distinct intermediate coupling element, separated and facing a primary guide circuit 17 in a first intermediate coupling tract and facing the secondary guide circuit 18 in a second intermediate coupling tract.

In accordance with possible embodiments, the coupling between the primary guide circuit 17 and the secondary guide circuit 18 may be obtained by means of a plurality of intermediate coupling elements coupled together, wherein at least one intermediate coupling element is distinct, separated and facing the primary guide circuit 17 in an intermediate coupling tract, and at least one other intermediate coupling element is distinct, separated and facing the secondary guide circuit 18 in another intermediate coupling tract.

In accordance with possible embodiments, the primary guide circuit 17 and/or the secondary guide circuit 18 may comprise a plurality of elements connected together to form the desired path.

According to possible embodiments, the elements present in the primary guide circuit 17 and/or in the secondary guide circuit 18 can be substantially configured as the elements of the guide circuit 12.

In accordance with possible embodiments, at least one primary guide circuit 17 may define a closed path.

According to possible embodiments, at least one primary guide circuit 17 may define an open path.

In accordance with possible embodiments, at least one secondary guide circuit 18 may define a closed path.

According to possible embodiments, at least one secondary guide circuit 18 may define an open path.

In accordance with possible embodiments, each of the transport units 14 may comprise a body 26 and a containment member 27 connected to the body 26 and configured to contain a number of absorbent sanitary articles.

According to possible embodiments, the containment member 27 may comprise means for defining the containment compartment of the absorbent sanitary articles so as to be able to group the absorbent sanitary articles in assemblies formed by a desired number of absorbent sanitary articles.

According to possible embodiments, the body 26 may be provided with a first magnetic element 28 configured to magnetically couple to the primary guide circuit 17 and with a second magnetic element 29, opposite to the first magnetic element, configured to magnetically couple to at least one of the secondary guide circuits 18.

In accordance with possible embodiments, the first magnetic element 28 and the second magnetic element 29 can be selectively activated so that the transport unit 14 is coupled to the primary guide circuit 17 or to one of the secondary guide circuits 18 in the coupling tract 13.

According to possible embodiments, the first magnetic element 28 and the second magnetic element 29 can be configured to advance said body 26 along the primary guide circuit 17 and the secondary guide circuit 18.

For example, the primary guide circuit 17 and the secondary guide circuit 18 may be configured in such a way that a variable magnetic field is generated, configured to magnetically couple with the first magnetic element 28 or the second magnetic element 29 in a tract of the primary guide circuit 17 or the secondary guide circuit 18, so that the body 26 can move along one of these guide circuits 17 and 18.

According to possible embodiments, the movement and/or stopping along the primary guide circuit 17 or the secondary guide circuit 18 may be obtained by magnetic movement means, aerostatic movement means, combinations thereof, or other means. For example, the movement may be substantially obtained as in linear magnetic motors.

According to possible embodiments, at least two of the secondary guide circuits 18 have their respective delivery areas 16 facing outwards of the secondary guide circuits 18 along two directions parallel to each other.

According to possible embodiments, at least two of the secondary guide circuits 18 have their respective delivery areas 16 facing outwards of the secondary guide circuits 18 along two directions perpendicular to each other.

According to possible embodiments, at least two secondary guide circuits 18 may have their respective delivery areas16 facing outwards of the secondary guide circuits 18 along two directions opposite to each other.

In accordance with possible embodiments, the primary guide circuit 17 may have the receiving area 15 facing towards the inside of the primary guide circuit 17 along a first direction D1 at which, during use, the absorbent sanitary articles are supplied, and at least one of the secondary guide circuits 18 may have the delivery area 16 facing outwards of the secondary guide circuit 18 along a second direction D2 opposite to the first direction D1.

According to possible embodiments, the primary guide circuit 17 may have the receiving area 15 where, during use, the absorbent sanitary articles are provided along a first direction D1 facing the inside of the primary guide circuit 17.

In accordance with possible embodiments, at least one of the secondary guide circuits 18 may have the delivery area 16 where, during use, the absorbent sanitary articles are delivered along a direction D2 facing outwards of the secondary guide circuit 18.

According to possible embodiments, the primary guide circuit 17 may comprise a second receiving area 115 functionally connected to the production machine M1 of absorbent sanitary articles.

This allows optimizing the delivery times of the absorbent sanitary articles by the production machine M1, since after having grouped the desired number of absorbent sanitary articles, and while these articles are delivered to the transport unit 14 in the receiving area 15, the production machine M1 groups the absorbent sanitary articles in the second receiving area 115 and then transfers them to the transport unit 14 without interrupting the production cycle.

The following embodiments, which are not a part of the invention, relate to a method for transporting absorbent sanitary articles by means of a transport apparatus 10.

According to possible embodiments, the transport method may comprise a supplying step of the absorbent sanitary articles from the production machine M1 to the transport units 14 in the receiving area 15, wherein each transport unit 14 carries a respective number of absorbent sanitary articles.

In accordance with possible embodiments, the transport method may comprise a setting step of the supply order of the absorbent sanitary articles transported by the transport units 14 for each of the packaging machines C1, C2, C3.

According to possible embodiments, the transport method may comprise a transporting step of absorbent sanitary articles by means of the transport units 14 towards the packaging machines C1, C2, C3.

In accordance with possible embodiments, the transport method may comprise a delivery step of the absorbent sanitary articles to the packaging machines C1, C2, C3 according to the respective set supply order.

According to possible embodiments, the supplying step may envisage providing at least two transport units 14 with a different number of absorbent sanitary articles.

According to possible embodiments, the supplying step may envisage providing at least two transport units 14 with a different type of absorbent sanitary articles.

According to possible embodiments, the transport apparatus 10 of absorbent sanitary articles may comprise:
- a guide assembly 11 wherein there is at least one receiving area 15 functionally connected to a production machine M of absorbent sanitary articles and at least one delivery area 16 functionally connected to a respective packaging machine C1; and
- a plurality of transport units 14, each one configured to receive the absorbent sanitary articles from the production machine M1 in the receiving area 15 and to transport them to the delivery area 16 along a desired path defined by the guide assembly 11.

According to possible embodiments, the guide assembly 11 may comprise an inlet tract 19 wherein the receiving area 15 is present, an outlet tract 20 wherein the delivery area 16 is present, an auxiliary inlet tract 21 wherein an auxiliary receiving area 22 is present, and a transit tract 23.

According to possible embodiments, the guide assembly 11 may comprise a first exchange assembly 24 connected to the transit tract 23, to the auxiliary inlet tract 21 and to the outlet tract 20, and defining a first connection path P1 between the transit tract 23 and the outlet tract 20, which includes the auxiliary inlet tract 21, and a second connection path P2 between the transit tract 23 and the outlet tract 20, which does not include the auxiliary inlet tract 21.

In accordance with possible embodiments, the guide assembly 11 may comprise a second exchange assembly 25 connected to the outlet tract 20, the inlet tract 19 and the transit tract 23, and defining a third connection path P3 between the outlet tract 20 and the transit tract 23, which includes the inlet tract 19 and a fourth path P4 between the outlet tract 20 and the transit tract 23, which does not include the inlet tract 19.

In accordance with possible embodiments, the guide assembly 11 may comprise a closed circuit 37 wherein the transit tract 23 and the outlet tract 20 are present.

In accordance with possible embodiments, at least one transport unit 14 may be configured to advance along the closed circuit 37 to proceed from the transit tract 23 to the outlet tract 20 to complete the second connection path P2.

In accordance with possible embodiments, at least one transport unit 14 may be configured to advance along the closed circuit 37 to proceed from the outlet tract 20 to the transit tract 23 to complete the fourth connection path P4.

According to possible embodiments, the first exchange assembly 24 may comprise a first distinct exchange element 38, separated and facing the closed circuit 37 and the auxiliary inlet tract 21, in a first coupling tract 113 and in a second coupling tract 213, respectively.

According to possible embodiments, the first exchange assembly 24 may comprise a second distinct exchange element 39, separated and facing the closed circuit 37 and the auxiliary inlet tract 21, in a third coupling tract 313 and in a fourth coupling tract 413, respectively.

According to possible embodiments, the second exchange assembly 25 may comprise a third distinct exchange element 40, separated and facing the closed circuit 37 and the inlet tract 19, in a fifth coupling tract 513 and in a sixth coupling tract 613, respectively.

According to possible embodiments, the second exchange assembly 25 may comprise a fourth distinct exchange element 41, separated and facing the closed circuit 37 and the inlet tract 19, in a seventh coupling tract 713 and in an eighth coupling tract 813, respectively.

In accordance with possible embodiments, at least one of the transport units 14 may be configured to pass from the closed circuit 37 to the first exchange element 38 at the first coupling tract 113.

In accordance with possible embodiments, at least one of the transport units 14 may be configured to pass from the first exchange element 38 to the auxiliary inlet tract 21 at the second coupling tract 213.

In accordance with possible embodiments, at least one of the transport units 14 may be configured to pass from the auxiliary inlet tract 21 to the second exchange element 39 at the fourth coupling tract 413.

In accordance with possible embodiments, at least one of the transport units 14 may be configured to pass from the second exchange element 39 to the closed circuit 37 at the third coupling tract 313.

In accordance with possible embodiments, at least one of the transport units 14 may be configured to pass from the closed circuit 37 to the third exchange element 40 at the fifth coupling tract 513.

In accordance with possible embodiments, at least one of the transport units 14 may be configured to pass from the third exchange element 40 to the inlet tract 19 at the sixth coupling tract 613.

In accordance with possible embodiments, at least one of the transport units 14 may be configured to pass from the inlet tract 19 to the fourth exchange element 41 at the eighth coupling tract 813.

In accordance with possible embodiments, at least one of the transport units 14 may be configured to pass from the fourth exchange element 41 to the closed circuit 37 at the seventh coupling tract 713.

According to possible embodiments, at least one transport unit 14 may be configured to pass in succession from the transit tract 23 of the closed circuit 37 to the first exchange element 38 to the auxiliary inlet tract 21 to the second exchange element 39 and to the outlet tract 20 of the closed circuit 37 to complete the first connection path P1.

According to possible embodiments, at least one transport unit 14 may be configured to pass in succession from the outlet tract 20 of the closed circuit 37 to the third exchange element 40 to the inlet tract 19 to the fourth exchange element 41 and to the transit tract 23 of the closed circuit 37 to complete the third connection path P3.

According to possible embodiments, the first exchange assembly 24 may comprise a first distinct open circuit 42, separated and facing the closed circuit in a first inlet coupling tract 43 and in a first outlet coupling tract 44 between which the auxiliary inlet tract 21 is comprised.

According to possible embodiments, the first open circuit 42 comprises the auxiliary inlet tract 21.

According to possible embodiments, at least one transport unit 14 may be configured to pass in succession from the transit tract 23 of the closed circuit 37 to the auxiliary inlet tract 21 comprised in the first open circuit 42, and from this to the outlet tract 20 of the closed circuit 37 to complete the first connection path P1.

According to possible embodiments, the second exchange assembly 25 may comprise a second distinct open circuit 45, separated and facing the closed circuit 37 in a second inlet coupling tract 46 and in a second outlet coupling tract 47 between which the inlet tract 19 is comprised.

According to possible embodiments, the second open circuit 45 comprises the inlet tract 19.

According to possible embodiments, at least one transport unit 14 may be configured to pass in succession from the outlet tract 20 of the closed circuit 37 to the inlet tract 19 comprised in the second open circuit 45, and from this to the transit tract 23 of the closed circuit 37 to complete the third connection path P3.

According to possible embodiments, at least one of the transport units 14 may be configured to pass from the closed circuit 37 to the first open circuit 42, and vice versa, at the first inlet coupling tract 43 and at the first outlet tract 44, respectively.

According to possible embodiments, at least one of the transport units 14 may be configured to pass from the closed circuit 37 to the second open circuit 45, and vice versa, at the second inlet coupling tract 46 and at the second outlet coupling tract 47, respectively.

According to possible embodiments, at least one transport unit 14 is configured to alternatively follow the first connection path P1 or the second connection path P2.

According to possible embodiments, at least one transport unit 14 is configured to alternatively follow the third connection path P3 or the fourth connection path P4.

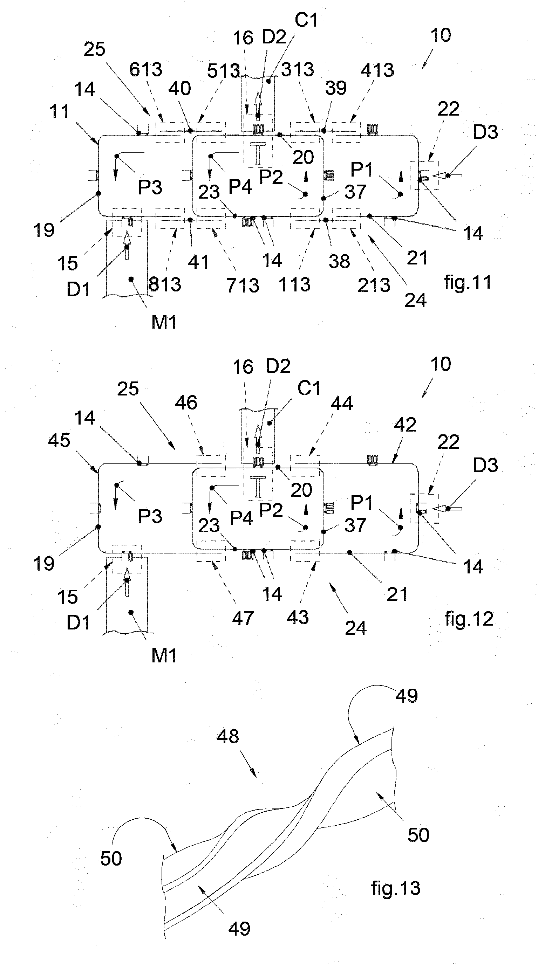

Both the configuration illustrated in Figure 11 and the configuration 12, as well as modifications or combinations of these configurations, allow at least one transport unit 14 to alternatively follow the first connection path P1 or the second connection path P2, and alternatively the third connection path P3 or the fourth connection path P4.

In accordance with possible embodiments, each of the transport units 14 may comprise a body 26 and a containment member 27 connected to the body 26 and configured to contain the absorbent sanitary articles.

According to possible embodiments, the body 26 may be provided with a first magnetic element 28 configured to magnetically couple to a portion of the guide assembly 11 and with a second magnetic element 29, opposite to the first magnetic element 28, configured to magnetically couple to another portion of the guide assembly 11.

In accordance with possible embodiments, the first magnetic element 28 and the second magnetic element 29 may be selectively activated so that the transport unit 14 is coupled to a portion of the guide assembly 11 in one of the coupling tracts 113, 213, 313, 413, 513, 613, 713, 813, 43, 44, 46, and 47.

According to possible embodiments, the containment member 27 may be connected to the body 26 by means of a connecting member 30 configured to rotate around a second rotation axis X2 perpendicular to the direction of movement D defined by the guide circuit 12.

In accordance with possible embodiments, the guide circuit 12 may comprise at least one spiral tract 48 wherein there is a first surface 49 and a second surface 50, opposite to the first surface 49, arranged along a spiral path, so that upon leaving the spiral path, the first surface 49 and the second surface 50 are inverted with each other.

According to possible embodiments, the receiving area 15 faces the inside of the guide assembly along a first direction D1, and the delivery area 16 faces the outside of the guide assembly 11 along a second direction D2 parallel to the first direction D1.

According to possible embodiments, at the receiving area 15, during use, the sanitary articles may be supplied towards the inside of the guide assembly 11 along a first direction D1.

According to possible embodiments, at the delivery area 16, during use, the sanitary articles may be supplied towards the outside of the guide assembly 11 along a second direction D2.

According to possible embodiments, at the auxiliary receiving area 22, during use, the sanitary articles may be supplied towards the inside of the guide assembly 11 along a third direction D3.

According to possible embodiments, the third direction D3 may be transversal to the first direction D1.

In accordance with possible embodiments, the guide assembly 11 may comprise a plurality of delivery areas 16 functionally connected to the packaging machine C1.

According to possible embodiments, at least two delivery areas 16 functionally connected to the packaging machine C1 may be oriented towards the outside of the guide assembly 11 along two parallel directions.

According to possible embodiments, the present invention also relates to a movable device 90 for transporting groups G1, G2, G3 of articles in a packaging plant 100.

For illustrative purposes only and non-limitative to this, in the following description we will refer to absorbent sanitary articles. It is understood that the movable device 90 can also be used to transport groups G1, G2, G3 of articles of other types in a packaging plant 100. For example, the articles may include books, CDs, DVDs, tools, various kinds of objects, bottles, containers, their combinations, or other articles.

In accordance with possible embodiments, the packaging plant 100 may comprise at least one production machine M1, M2, M3 of groups G1, G2, G3 of articles and at least one packaging machine C1, C2, C3 configured to form a desired combination of groups G1, G2, G3 of articles.

According to possible embodiments, the movable device 90 may comprise a frame 51 provided with movement means 52 configured to move the frame 51 in the packaging plant 100 at least from the production machine M1, M2, M3 to the packaging machine C1, C2, C3.

In accordance with possible embodiments, the movement means 52 may comprise wheels, tracks, actuators, motors, transmissions, gears, transmission belts, balls, rollers, and their combinations, or other suitable means for moving the frame 51 in the packaging plant 100.

According to possible embodiments, the movable device 90 may comprise a guide circuit 12 coupled to the frame 51, and at least one transport unit 14.

In accordance with possible embodiments, the transport units may comprise a body 26 and a containment member 27 connected to the body 26 and configured to contain at least one of the groups G1, G2, G3 of articles.

According to possible embodiments, the body 26 may be provided with a first magnetic element 28 configured to magnetically couple to the guide circuit 12, so that, during use, the body 26 is in the desired position along the guide circuit 12.

In accordance with possible embodiments, the body 26 may be configured to position itself in the desired position along the guide circuit 12.

According to possible embodiments, the transport unit 14 may be provided with a second magnetic element 29, opposite to the first magnetic element 28, configured to magnetically couple to another guide circuit 12 distinct and separated from said guide circuit 12.

In accordance with possible embodiments, the other guide circuit 12 may be present in the packaging plant 100.

According to possible embodiments, the other guide circuit 12 may be present in another movable device 90.

In accordance with possible embodiments, the first magnetic element 28 and the second magnetic element 29 may be selectively activated so that the transport unit is coupled to the guide circuit 12, or to the other guide circuit 12 at a coupling tract 13 wherein, during use, the guide circuit 12 and the other guide circuit 12 are facing and separated from each other.

According to possible embodiments, the movable device 90 may comprise at least two distinct guide circuits 12, separated and facing each other in respective coupling tracts 13.

In accordance with possible embodiments, the movable device 90 may comprise a lifting member 53 configured to carry the guide circuit 12 from a first height to a second height different from the first height.

According to possible embodiments, the movable device 90 may comprise a communication unit 54 configured for receiving and transmitting a signal with a control and command unit 36 configured to control the movable device 90 and/or other movable devices 90 present in the packaging plant 100.

In accordance with possible embodiments, the movable device 90 may comprise a rechargeable electric power supply unit 55 configured to power the movable device 90.

For example, the rechargeable electric power supply unit 55 may power the movement means 52 and/or other components of the movable device 90.

According to possible embodiments, the present invention also relates to a packaging plant 100 of groups G1, G2, G3 of articles, comprising at least one production machine M1, M2, M3 of groups G1, G2, G3 of articles and at least one packaging machine C1, C2, C3 configured to form a desired combination of groups G1, G2, G3 of articles.

In accordance with possible embodiments, the packaging plant 100 may comprise a plurality of movable devices 90 as in any of the possible embodiments of the present invention.

According to possible embodiments, the packaging plant 100 may comprise a guide circuit 12 functionally coupled to at least one production machine M1, M2, M3, so that, during use, the transport units 14 can be positioned at the production machine M1, M2, M3.

This aspect allows maintaining the production continuity of the production machine M1, M2, M3 which, after completing the transfer to a movable device 90, does not need to wait for another movable device 90 to deliver the articles to the latter.

This aspect allows the production machine M1, M2, M3 to transfer the produced articles to the transport units 14 present in the guide circuit 12 functionally coupled to the production machine M1, M2, M3.

In this way, the movable devices 90 may position themselves at the guide circuit 12 functionally coupled to the production machine M1, M2, M3, so as to allow the exchange of the transport units 14 with the articles from the guide circuit 12 functionally coupled to the production machine M1, M2, M3 to the guide circuit 12 of the movable device 90, and possibly the simultaneous exchange of the empty or partially empty transport units 14 from the guide circuit 12 of the transport unit 14 to the guide circuit 12 coupled to the production machine M1, M2, M3.

In accordance with possible embodiments, the packaging plant 100 may comprise a guide circuit 12 functionally coupled to at least two packaging machines C1, C2, C3, so that, during use, the transport units 14 can be positioned at any of these packaging machines C1, C2, C3.

According to possible embodiments, the packaging plant 100 may comprise a control and command unit 36 configured to control the plurality of movable devices 90 in a coordinated manner so as to form the desired combination of groups G1, G2, G3 of articles in the packaging machine C1, C2, C3.

In accordance with possible embodiments, the packaging plant 100 may comprise a charging station 56 wherein there is at least one charging device 57 configured to recharge one or more rechargeable electric power supply units 55 of the movable devices 90.

According to possible embodiments, at least one movable device 90 may be provided with transfer means configured to transfer the articles from the transport units 14 to the packaging machines C1, C2, C3.

According to possible embodiments, at least one movable device 90 may be provided with transfer means configured to transfer the articles from the production machines M1, M2, M3 to the transport units 14.

According to possible embodiments, at least one movable device 90 may be provided with transfer means configured to transfer the articles from the transport units 14 of the movable device 90 to the transport units 14 of another movable device 90.

According to possible embodiments, at least one movable device 90 may be provided with transfer means configured to transfer the articles from the transport units 14 of the movable device 90 to the transport units 14 of the guide circuit 12 present in the packaging plant 100. For example, the transfer means may comprise an articulated robot, an anthropomorphic robot, or other similar means.

## Claims

1. A movable device for transporting groups (G1, G2, G3) of articles in a packaging plant (100) comprising at least one production machine (M1, M2, M3) of said groups (G1, G2, G3) of articles and at least one packaging machine (C1, C2, C3) configured to form a desired combination of said groups (G1, G2, G3) of articles, wherein said movable device (90) comprises a frame (51) provided with movement means (52) configured to move said frame (51) in said packaging plant (100) at least from said production machine (M1, M2, M3) to said packaging machine (C1, C2, C3), wherein said movable device (90) comprises a guide circuit (12) coupled to said frame (51), and at least one transport unit (14) comprising a body (26) and a containment member (27) connected to said body (26) and configured to contain at least one of said groups (G1, G2, G3) of articles, wherein said body (26) is provided with a first magnetic element (28) configured to magnetically couple to said guide circuit (12), so that, during use, said body (26) is in the desired position along said guide circuit (12),
wherein said transport unit (14) is provided with a second magnetic element (29), opposite to said first magnetic element (28), configured to magnetically couple to another guide circuit (12) which is distinct and separated from said guide circuit (12) and present in said packaging plant (100), or present in another movable device (90), wherein said first magnetic element (28) and said second magnetic element (29) are selectively activable so that said transport unit (14) is coupled to said guide circuit (12) or to said other guide circuit (12) at a coupling tract (13) wherein, during use, said guide circuits (12) face each other.

2. A movable device as in claim 1, **characterized in that** it comprises at least two guide circuits (12) which are distinct, separated and facing each other at respective coupling tracts (13).

3. A movable device as in claims 1 or 2, **characterized in that** it comprises a lifting member (53) configured to carry said guide circuit (12) from a first height to a second height, different from said first height.

4. A movable device as in any of the preceding claims, **characterized in that** it comprises a communication unit (54) configured to receive and transmit a signal with a control and command unit (36) configured to control said movable device (90) and/or other movable devices (90) present in said packaging plant (100).

5. A movable device as in any of the preceding claims, **characterized in that** it comprises a rechargeable electric power supply unit (55) configured to electrically supply said movable device (90).

6. A packaging plant (100) of groups (G1, G2, G3) of articles comprising at least one production machine (M1, M2, M3) of said groups (G1, G2, G3) of articles and at least one packaging machine (C1, C2, C3) configured to form a desired combination of said groups (G1, G2, G3) of articles, **characterized in that** it comprises a plurality of movable devices (90) as claimed in any of the preceding claims.

7. A plant as in claim 6, **characterized in that** it comprises a guide circuit (12) functionally coupled to at least one production machine (M1, M2, M3), so that, during use, said transport units (14) are positionable at said production machine (M1, M2, M3).

8. A plant as in claims 6 or 7, **characterized in that** it comprises a control and command unit (36) configured to control said plurality of movable devices (90) in a coordinated manner so as to form the desired combination of said groups (G1, G2, G3) of articles in said packaging machine (C1, C2, C3).

9. A plant as in any claim from 6 to 8, **characterized in that** it comprises a recharging station (56) wherein there is at least one recharging device (57) configured to recharge one or more rechargeable electric power supply units (55) of said movable devices (90).

## Patentansprüche

1. Bewegliche Einrichtung zum Transportieren von Gruppen (G1, G2, G3) von Gegenständen in einer Verpackungsanlage (100), die mindestens eine Produktionsmaschine (M1, M2, M3) der Gruppen (G1, G2, G3) von Gegenständen und mindestens eine Verpackungsmaschine (C1, C2, C3) umfasst, die dafür konfiguriert ist, eine gewünschte Kombination der Gruppen (G1, G2, G3) von Gegenständen zu bilden, wobei die bewegliche Einrichtung (90) einen Rahmen (51) umfasst, der mit Bewegungsmitteln (52) versehen ist, die dafür konfiguriert sind, den Rahmen (51) in der Verpackungsanlage (100) zumindest von der Produktionsmaschine (M1, M2, M3) zu der Verpackungsmaschine (C1, C2, C3) zu bewegen, wobei die bewegliche Einrichtung (90) einen mit dem Rahmen (51) gekoppelten Führungskreis (12) und mindestens eine Transporteinheit (14) umfasst, die ein Gehäuse (26) und ein Aufnahmeelement (27) umfasst, das mit dem Gehäuse (26) verbunden und dafür konfiguriert ist, mindestens eine der Gruppen (G1, G2, G3) von Gegenständen aufzunehmen, wobei das Gehäuse (26) mit einem ersten magnetischen Element (28) versehen ist, das für magnetische Kopplung mit dem Führungskreis (12) konfiguriert ist, so dass sich im Betrieb das Gehäuse (26) in der gewünschten Position entlang des Führungskreises (12) befindet,
wobei die Transporteinheit (14) mit einem zweiten magnetischen Element (29) gegenüber dem ersten magnetischen Element (28) versehen ist, das für magnetische Kopplung mit einem anderen Führungskreis (12) konfiguriert ist, der zu dem Führungskreis (12) eigenständig und von diesem getrennt in der Verpackungsanlage (100) oder in einer anderen beweglichen Einrichtung (90) vorhanden ist, wobei das erste magnetische Element (28) und das zweite magnetische Element (29) selektiv so aktivierbar sind, dass die Transporteinheit (14) auf einem Kopplungsabschnitt (13) mit dem Führungskreis (12) oder mit dem anderen Führungskreis (12) gekoppelt wird, wobei im Betrieb die beiden Führungskreise (12) einander zugewandt sind.

2. Bewegliche Einrichtung nach Anspruch 1, **dadurch gekennzeichnet, dass** sie mindestens zwei Führungskreise (12) umfasst, die eigenständig und getrennt auf jeweiligen Kopplungsabschnitten (13) einander zugewandt sind.

3. Bewegliche Einrichtung nach Anspruch 1 oder 2, **dadurch gekennzeichnet, dass** sie ein Hubelement (53) umfasst, das dafür konfiguriert ist, den Führungskreis (12) von einer ersten Höhe auf eine von der ersten Höhe verschiedene zweite Höhe zu tragen.

4. Bewegliche Einrichtung nach einem der vorhergehenden Ansprüche, **dadurch gekennzeichnet, dass** sie eine Kommunikationseinheit (54) umfasst, die dafür konfiguriert ist, mit einer Steuer- und Befehlseinheit (36), die dafür konfiguriert ist, die bewegliche Einrichtung (90) und/oder andere in der Verpackungsanlage (100) vorhandene bewegliche Einrichtungen (90) zu steuern, ein Signal zu empfangen und zu übertragen.

5. Bewegliche Einrichtung nach einem der vorhergehenden Ansprüche, **dadurch gekennzeichnet, dass** sie eine wiederaufladbare Stromversorgungseinheit (55) umfasst, die dafür konfiguriert ist, die bewegliche Einrichtung (90) mit Strom zu versorgen.

6. Verpackungsanlage (100) für Gruppen (G1, G2, G3) von Gegenständen, die mindestens eine Produktionsmaschine (M1, M2, M3) der Gruppen (G1, G2, G3) von Gegenständen und mindestens eine Verpackungsmaschine (C1, C2, C3) umfasst, die dafür konfiguriert ist, eine gewünschte Kombination der Gruppen (G1, G2, G3) von Gegenständen zu bilden, **dadurch gekennzeichnet, dass** sie eine Vielzahl beweglicher Einrichtungen (90) nach einem der vorhergehenden Ansprüche umfasst.

7. Anlage nach Anspruch 6, **dadurch gekennzeichnet, dass** sie einen funktionell mit der mindestens einen Produktionsmaschine (M1, M2, M3) gekoppelten Führungskreis (12) umfasst, so dass im Betrieb die Transporteinheiten (14) an der Produktionsmaschine (M1, M2, M3) positionierbar sind.

8. Anlage nach Anspruch 6 oder 7, **dadurch gekennzeichnet, dass** sie eine Steuer- und Befehlseinheit (36) umfasst, die dafür konfiguriert ist, die Vielzahl beweglicher Einrichtungen (90) koordiniert zu steuern, um die gewünschte Kombination der Gruppen (G1, G2, G3) von Gegenständen in der Verpackungsmaschine (C1, C2, C3) zu bilden.

9. Anlage nach einem der Ansprüche 6 bis 8, **dadurch gekennzeichnet, dass** sie eine Ladestation (56) umfasst, wobei mindestens eine Ladeeinrichtung (57) vorhanden ist, die dafür konfiguriert ist, eine oder mehrere wiederaufladbare Stromversorgungseinheiten (55) der beweglichen Einrichtungen (90) erneut zu laden.

## Revendications

1. Dispositif mobile pour transporter des groupes (G1, G2, G3) d'articles dans une installation d'emballage (100) comprenant au moins une machine de production (M1, M2, M3) desdits groupes (G1, G2, G3) d'articles et au moins une machine d'emballage (C1, C2, C3) configurée pour former une combinaison souhaitée desdits groupes (G1, G2, G3) d'articles, où ledit dispositif mobile (90) comprend un cadre (51) pourvu de moyens de déplacement (52) configurés pour déplacer ledit cadre (51) dans ladite installation d'emballage (100), au moins de ladite machine de production (M1, M2, M3) à ladite machine d'emballage (C1, C2, C3), où ledit dispositif mobile (90) comprend un circuit de guidage (12) couplé audit cadre (51), et au moins une unité de transport (14) comprenant un corps (26) et un organe de retenue (27) relié audit corps (26) et configuré pour contenir au moins l'un desdits groupes (G1, G2, G3) d'articles, où ledit corps (26) est pourvu d'un premier élément magnétique (28) configuré pour se coupler magnétiquement audit circuit de guidage (12), de sorte que, lors de l'utilisation, ledit corps (26) soit dans la position souhaitée le long dudit circuit de guidage (12), où ladite unité de transport (14) est pourvue d'un deuxième élément magnétique (29), opposé audit premier élément magnétique (28), configuré pour se coupler magnétiquement à un autre circuit de guidage (12) qui est distinct et séparé dudit circuit de guidage (12) et présent dans ladite installation d'emballage (100), ou présent dans un autre dispositif mobile (90), où ledit premier élément magnétique (28) et ledit deuxième élément magnétique (29) peuvent être sélectivement activés de sorte que ladite unité de transport (14) soit couplée audit circuit de guidage (12) ou audit autre circuit de guidage (12) au niveau d'un tronçon de couplage (13) où, lors de l'utilisation, lesdits circuits de guidage (12) se font face.

2. Dispositif mobile selon la revendication 1, **caractérisé en ce qu'**il comprend au moins deux circuits de guidage (12) qui sont distincts, séparés et se font face au niveau de tronçons de couplage respectifs (13).

3. Dispositif mobile selon la revendication 1 ou 2, **caractérisé en ce qu'**il comprend un organe de levage (53) configuré pour porter ledit circuit de guidage (12) d'une première hauteur à une deuxième hauteur, différente de ladite première hauteur.

4. Dispositif mobile selon l'une des revendications précédentes, **caractérisé en ce qu'**il comprend une unité de communication (54) configurée pour recevoir et transmettre un signal avec une unité de contrôle et de commande (36) configurée pour contrôler ledit dispositif mobile (90) et/ou d'autres dispositifs mobiles (90) présents dans ladite installation d'emballage (100).

5. Dispositif mobile selon l'une des revendications précédentes, **caractérisé en ce qu'**il comprend une unité d'alimentation électrique rechargeable (55) configurée pour alimenter électriquement ledit dispositif mobile (90).

6. Installation d'emballage (100) de groupes (G1, G2, G3) d'articles comprenant au moins une machine de production (M1, M2, M3) desdits groupes (G1, G2, G3) d'articles et au moins une machine d'emballage (C1, C2, C3) configurée pour former une combinaison souhaitée desdits groupes (G1, G2, G3) d'articles, **caractérisée en ce qu'**elle comprend une pluralité de dispositifs mobiles (90) tel que revendiqué dans l'une des revendications précédentes.

7. Installation selon la revendication 6, **caractérisée en ce qu'**elle comprend un circuit de guidage (12) couplé de manière fonctionnelle à au moins une machine de production (M1, M2, M3), de sorte que, lors de l'utilisation, lesdites unités de transport (14) puissent être positionnées au niveau de ladite machine de production (M1, M2, M3).

8. Installation selon la revendication 6 ou 7, **caractérisée en ce qu'**elle comprend une unité de contrôle et de commande (36) configurée pour contrôler ladite pluralité de dispositifs mobiles (90) de façon coordonnée de manière à former la combinaison souhaitée desdits groupes (G1, G2, G3) d'articles dans ladite machine d'emballage (C1, C2, C3).

9. Installation selon l'une des revendications 6 à 8, **caractérisée en ce qu'**elle comprend un poste de recharge (56) dans lequel il y a au moins un dispositif de recharge (57) configuré pour recharger une ou plusieurs unités d'alimentation électrique rechargeables (55) desdits dispositifs mobiles (90).
